# EUROPEAN PATENT APPLICATION

(11) **EP 1 138 777 A2**
(43) Date of publication of application: **04.10.2001**
(21) Application number: 01302601.8
(22) Date of filing: 21.03.2001
(51) Int. Cl.: C12Q 1/25, C12Q 1/42, C12Q 1/02, B01L 3/00

(54) **Temperature history indicator**

(30) Priority: 23.03.2000 JP 2000082010
(71) Applicant: KONICA CORPORATION, Tokyo (JP)
(72) Inventor: Uemura, Morito, Hino-shi, Tokyo (JP)
(74) Representative: Eddowes, Simon

(57) **Abstract**

A temperature history indicator in which a reaction is initiated by blending a plurality of separate reagents is disclosed. The temperature history indicator characterized in that an enzyme and an enzyme substrate are employed as said reagents; said enzyme and said enzyme substrate undergo color reaction; said color reaction is carried out in the specified temperature range so as to form a specified color. The disposable temperature history indicator which can be directly attached onto food packages and is useful for the management as well as the maintenance in food sanitation in the temperature range which is suitable for the growth of bacteria.

## Description

### FIELD OF THE INVENTION

The present invention relates to a temperature history indicator.

### BACKGROUND OF THE INVENTION

Heretofore, as devices to which techniques to record the variation of temperature over an elapse of time are applied, automatic recording thermometers, in which an electrical thermal sensor and a memory medium are combined, have been employed. A portable device, comprised of an electrical thermal sensor and an IC, is also employed, and the resultant data can be downloaded to computers.

Recently, the necessity of managing the temperature of a perishable food during the storage or transportation thereof is increased. For example, it is necessary that the perishable food is kept at a low temperature through the period from the production to the arrival to the consumer's hand. For such the purpose, the food is distributed under a cooled or frozen condition. When the perishable food is exposed to an ordinary temperature for a certain period, a dangerousness of proliferation of a microorganism such as a bacterium is occurred. Accordingly, it is necessary to prevent passing the perishable food which has been stood at a temperature at which the microorganism is proliferated for a certain period to the consumer's hand.

It is very difficult, however, to judge the history of the temperature management of the perishable food according to the appearance thereof. Japanese Patent Publication for Public Inspection, hereinafter referred to JP O.P.I., No. 11-194053 discloses a temperature history indicator utilizing the diffusion of a dye having a temperature independency for indication the temperature history within the temperature range in which the dangerousness of the microorganism proliferation is occurred. Furthermore, a temperature history indicator utilizing a thermally decomposable chelate dye is proposed in JP O.P.I. 9-264797. US Patent No. 5,964,181 proposes a temperature history indicator composed of the combination of an organic compound having a specific melting point and a microporous membrane.

It is necessary to check the history of temperature at which the perishable food is stood by the manufacturer, distributor, custodian and seller at various steps such as the production, distribution and storage of the perishable food. It is more preferable that the temperature history of each of the individual perishable food can be judged by the consumer at the storefront. The dangerousness of taking the perishable food with the proliferated microorganism by the consumer can be further reduced by giving the discriminatablity by consumer to the temperature history of the individual food. Thus it is expected that the safeness and the sanitary condition of the perishable food can be considerably raised. However, it has be practically difficult to provide the temperature history indicator to the individual perishable food since the known temperature history indicators such as those described in the foregoing publications are expensive and the cost of the food is raised.

Accordingly, an temperature history indicator which is cheap in the coast, easily usable, preferably disposable, and directly attachable on the individual food is strongly demanded, by which the temperature history of the food can be directly recognized by the consumer.

Moreover, it is expected that such the temperature history indicator is applied not only to the perishable food but in the various fields since indicator can be applied to various things which are required to be managed regarding the temperature.

### SUMMARY OF THE INVENTION

The object of the invention is to provide a temperature history indicator which is cheap in the cost, easily usable and disposable. The further object of the invention is to provide a temperature history indicator capable of providing, for example, capable of directly attaching, to the individual food, by which the consumer can be directly recognize the temperature history of the food. Moreover, the object of the invention is to provide a temperature history indicator useful for managing and keeping the sanitary condition of food within the range suitable for proliferation of a microorganism.

The invention and its preferable embodiments are described.
1. A temperature history indicator comprising an enzyme or a microorganism and an enzyme substrate and a container to contain the enzyme or the microorganism and the enzyme substrate,
   wherein the enzyme substrate is separated from the enzyme or the microorganism,
   the enzyme substrate reacts with the enzyme or the microorganism by mixing the enzyme substrate with the enzyme or the microorganism,
   a specific color is created in response to the reaction, and the reaction proceeds at the specified temperature range.
2. The temperature history indicator of item 1, wherein the reaction proceeded by mixing the enzyme substrate with the enzyme or the microorganism is color creating reaction.
3. The temperature history indicator of item 1, wherein the indicator comprises a pH indicator, the reaction proceeded by mixing the enzyme substrate with the enzyme or the microorganism is a reaction to induce pH variation, and the pH indicator creates color in response to the pH variation.
4. The temperature history indicator of item 1, wherein the specified temperature range falls within the range of 0 to 80 °C.
5. The temperature history indicator of item 4, wherein the specified temperature range falls within the range of 4 to 65 °C.
6. The temperature history indicator of item 1, wherein the enzyme is a thermoduric enzyme which is not modified at 50° C or the microorganism is a thermoduric microorganism which does not perish at 50° C.
7. The temperature history indicator of item 1, wherein the enzyme is an enzyme derived from a thermophilic bacterium or the microorganism is a thermophilic bacterium.
8. The temperature history indicator of item 7, wherein the enzyme is alanine dehydrogenase derived from bacillus stearothermophilus, alanine racemase derived from bacillus stearothermophilus, or formate dehydrogenase derived from a microorganism.
9. The temperature history indicator of item 7, wherein the microorganism is bacillus stearothermophilus.
10. The temperature history indicator of item 1, wherein the container comprises a first containing portion containing the enzyme or the microorganism and a second containing portion containing the enzyme substrate, and the container constituted so that the enzyme or the microorganism can be mixed with the enzyme substrate by pressing the first or second containing portion.
11. The temperature history indicator of item 10, wherein the first containing portion or the second containing portion is visibly observable..
12. The temperature history indicator of item 10, wherein the enzyme or microorganism is dissolved or dispersed in a liquid.
13. The temperature history indicator of item 10, wherein the enzyme substrate is dissolved or dispersed in a liquid.
14. The temperature history indicator of item 10, wherein the indicator comprises a adhesive portion by which the indicator is adhered on another thing.

The other embodiment will be described.
(1) In a temperature history indicator in which recording is initiated upon blending a plurality of separate reagents, a temperature history indicator wherein an enzyme and an enzyme substrate are employed as said reagents; said enzyme and said enzyme substrate undergo color reaction; said color reaction proceeds only at the specified temperature; and a specific color is created.
(2) In a temperature history indicator in which recording is initiated upon blending a plurality of separate regents, a temperature history indicator wherein an enzyme as well as an enzyme substrate is employed as said reagents; reaction between said enzyme and said enzyme substrate results in pH variation; further, said reaction proceeds at the specified temperature; and said pH variation is detected using a pH indicator.
(3) In a temperature history indicator in which recording is initiated upon blending a plurality of separate reagents, a temperature history indicator wherein a microorganism, an enzyme substrate, and a medium are employed as said reagents; said microorganism increases in the range of a specific temperature; the enzyme in said microorganism and said enzyme substrate undergo color reaction to indicate a specific color.
(4) In a temperature history indicator in which recording is initiated upon blending a plurality of separate reagents, a temperature history indicator wherein a microorganism and a medium are employed as said reagents; said microorganism grows in the range of specified temperature; the pH varies in accordance with said growth; and said pH variation is detected using a pH indicator.
(5) The temperature history indicator described in any one of (1) through (4) above, wherein a specified temperature range falls within the range of 0 to 80 °C.
(6) The temperature history indicator described in any one of (1) through (4) above, wherein a specified temperature range falls within the range of 5 to 65 °C.
(7) The temperature history indicator described in (1) or (2) above, wherein an enzyme and an enzyme substrate are arranged in the same packaging unit, and said enzyme and said enzyme substrate are blended.
(8) The temperature history indicator described in (3) above, wherein at least one of a microorganism, an enzyme substrate or a medium is individually arranged in the same packaging unit, and said microorganism is blended with said enzyme substrate or said medium.
(9) The temperature history indicator described in (4) above, wherein each of a microorganism, and a medium is arranged in the same packaging unit and said microorganism is blended with said medium.
(10) The temperature history indicator described in (1), (2), or (3) above, wherein at least one of an enzyme or an enzyme substrate is a liquid reagent, and by pressing the portion of said liquid reagent, blending with the other reagent is achieved.
(11) The temperature history indicator described in (3) or (8) above, wherein at least one selected from a microorganism, an enzyme substrate, and a medium is a liquid reagent, and by pressing the portion of said liquid reagent, blending with the other reagents is achieved.
(12) The temperature history indicator described in (4) or (9) above, wherein either a microorganism or a medium is a liquid reagent, and by pressing the portion of said liquid reagent, blending with the other reagent is carried out.
(13) The temperature history indicator described in (1) or (3) above, wherein dye B is previously incorporated which exhibits different hue from dye A which is formed through reaction between an enzyme and an enzyme substrate.
(14) The temperature history indicator described in (10) above, wherein each of an enzyme and an enzyme substrate is placed in a molded film; at the start of use, said molded film which separates said enzyme from said enzyme substrate is peeled away through pressing; and said enzyme and said enzyme substrate are blended.
(15) The temperature history indicator described in 11. above, wherein a microorganism, an enzyme substrate, or a medium is placed in a molded film; at the start of use, said molded film which separates said microorganism from said enzyme substrate or a medium is peeled away through pressing; and said microorganism and said enzyme substrate or said medium are blended.
(16) The temperature history indicator described in (12) above, wherein a microorganism and a medium are placed in a molded film; at the start of use, said molded film which separates said microorganism from said medium is peeled away through pressing; and said microorganism and said medium are blended.

### BRIEF DESCRIPTION OF THE DRAWINGS

Fig. 1(a) is a schematic view and a cross-sectional view of a development use sheet type temperature history indicator, prior to use.

Fig. 1(b) is a schematic view of a development use sheet type temperature history indicator at the beginning of use.

Fig. 1(c) is a schematic view of a development use sheet type temperature history indicator after temperature history use.

Fig. 2(a) is a schematic view and a cross-sectional view of a blister packaging container type temperature history indicator prior to use.

Fig. 2(b) is a schematic view of a blister packaging container type temperature history indicator at the beginning of use.

Fig. 2(c) is a schematic view of a blister packaging container type temperature history indicator after temperature history use and a schematic view of a color sample for visual judgment.

### DETAILED DESCRIPTION OF THE PREFERRED EMBODIMENT.

The present invention will now be detailed.

The present invention relates to a temperature history indicator, and preferably relates to a temperature history indicator which makes it possible to visually judge the temperature history and is suitable for showing the temperature history of food. In the distribution as well as the storage of food, in order to minimize the growth of microorganisms, high or low temperature management is carried out. The present invention is to provide a temperature history indicator which exhibits excellent correlation with temperature and time, which are factors of the growth of microorganisms. The growth of microorganisms is affected by factors other than the temperature and time, such as, for example, nutrients necessary for growth, moisture activity, growth inhibiting substances, and the like. Therefore, said correlation is not held for all conditions. However, the present invention makes it possible to carry out simple recording of the variation of temperature over an elapse of time. Thus, it is possible to employ the temperature history indicator of the present invention not only for the indication of the growth of microorganisms but also for the indication of the shelf life of packaged food items.

The temperature history indicator according to the invention comprises a packaging container for including an enzyme or an microorganism and an enzyme substrate, or an enzyme or a microorganism and an enzyme substrate and a container to contain these. In the container, the enzyme or the microorganism is separated from the enzyme substrate and a chemical reaction is occurred when the enzyme or the microorganism is mixed with the enzyme substrate so as to form a specific color. The chemical reaction is occurred within a prescribed temperature range.

The formation of the color in the invention includes not only coloring of a colorless substance but also increasing in the color density of a colored substance and changing in the hue of a colored substance.

The color is formed accompanied with the occurring of the chemical reaction when the temperature history indicator having such the constitution is stood within the prescribed temperature range after the enzyme or the microorganism is mixed with the enzyme substrate of the temperature history indicator. The standing time of the temperature history indicator within the prescribed temperature range can be easily judged by visually observing the color forming situation since the color density is increased or the hue of the color is varied accompanied with the length of the standing time within the prescribed temperature range. The indicator can be suitably applied as a temperature history indicator when the temperature range is set at a range in which the microorganism proliferate. The constitution of the indicator can be made simple by the use of the enzyme or the microorganism and the enzyme substrate, and the indicator can be ceaply produced.

The chemical reaction occurred by mixing the enzyme or the microorganism, and the enzyme substrate may be a color-formation reaction, or the reaction may be a pH varying-reaction so as to form a color of a pH indicating agent by the variation of the pH value. The temperature history indicator include a pH indicating reagent when the chemical reaction is the pH varying-reaction.

The prescribed temperature range in which the reaction is occurred by mixing the enzyme or the microorganism and the enzyme substrate is preferably from 0° to 80° C. In the case of the temperature history indicator for managing the temperature history of an ordinary perishable food, the temperature range may be set according to the using purpose of the indicator, and the temperature range of preferably from 4° C to 65° C, more referably 5° C to 60° C, and particularly referably 15° C to 60° C. Such the temperature range is preferable since it is easily judged that the temperature indicator is stood or not at a temperature at which the microorganism proliferates in the perishable food.

Preferable examples of the enzyme and the microorganism are described below. Among them, a thermoduric enzyme which is not modified at 50° C or a thermoduric microorganism which does not perish at 50° C. An enzyme which is not modified at 70° C or a microorganism which does not perish at 70° C, are more preferable. Preferable examples of the enzyme and the microorganism are described below.

It is preferable that the enzyme is an enzyme derived from a thermophilic bacterium, and the microorganism is a thermophilic bacterium. For example, a thermophilic bacterium of thermophilic bacillus or a enzyme derived from such the thermophilic bacterium is preferable. In concrete, alanine dehydrogenase derived from bacillus stearothermophilus, alanine racemase derived from bacillus stearothermophilus, and formate dehydrogenase derived from a microorganism are preferred. Besides, a thermoduric microorganism such as bacillus stearothermophilus.

The preferable combination of the enzyme and the substrate is listed.
1) Diaphorase and NADH⁺ Tetrazorium Group
2) β-Galactosidase and X-Gal, naphthalene
3) Alkali phosphatase and X-phosphate, naphthalene
4) β-Glucuronidase and X-Gluc

The reactions of the combinations of 1) and 3) mentioned above are pH variation reaction whereby the pH indicator colors or varies color. By the reaction of 2) and 4) the substrate is hydrolysed to prepare a colored dye.

The enzyme or microorganism each may be dissolved or dispersed in a liquid and the enzyme substrate may be dissolved or dispersed in a liquid. When the pH indicating agent is used, the pH indicating agent may be contained in the liquid containing the enzyme substrate or the liquid containing the enzyme or the microorganism.

When the microorganism is used, the temperature history indicator preferably contains culture medium. The culture medium may contains the enzyme substrate or the pH indicating agent. The culture medium includes a natural medium such as gelatin in which nutrient is pepton extract, a synthetic medium such as Simmonds citric acid medium, and a semi-synyhetic medium such as Mueller-Hiniton medium.

A dye having a color different from that formed by the chemical reaction may be previously contained in the temperature history indicator. For example, it is possible that the dye is contained in the liquid containing the enzyme or the microorganism or in the liquid containing the enzyme substrate.

The packaging container preferably has a first containing portion containing the enzyme or the microorganism and a second containing portion containing the enzyme substrate. It is preferable that the packaging container constituted so that the enzyme or the microorganism can be mixed with the enzyme substrate by pressing the first or second containing portion. When the pH indicating agent is used, the pH indicating agent is preferably contained in any one of the first and second containing portions. The first or second containing portion may be paper, cloth or nonwoven fabric in which the liquid containing the enzyme or the microorganism is immersed.

The first or second containing portion is preferably visibly observable. For example, a part or all of the first or second containing portion is preferably covered with a transparent material.

The temperature history indicator preferably has a adhesive portion by which the indicator can be adhered on another thing. The indicator preferably has a seal portion, an adhesive tape potion or a past portion.

Moreover, a color sample showing the relation between the color formation and the temperature history is preferably attached to the temperature history indicator.

The inventors of the present invention have been involved in the development of a temperature history indicator in which enzyme reaction is initiated by blending a plurality of separate reagents. In each case, in which said reagents are composed of an enzyme and an enzyme substrate and in which said reagents are composed of microorganisms and a medium containing an unspecified enzyme substrate, by preparing said case so as to result in coloration in the specified temperature range, the inventors have successfully developed a temperature history indicator which exhibits excellent correlation of temperature during the growth of bacteria over time.

Due to insufficient temperature management of food, toxic bacteria grow to often result in food poisoning. Utilizing the temperature history indicator may prevent such food poisoning. Under such a background as described above, demanded has been a disposable temperature history indicator which is less expensive but still simple, and can be attached to food packaging units. Then the inventors of the present invention have succeeded to meet requirements (a) through (d) listed below:
(a) to be applicable to the temperature range of 5 to 65 °C and to exhibit the maximum detective sensitivity of said indictor at about 35 °C, at which toxic bacteria grow rapidly,
(b) to result in color variation and preferably hue variation so that a temperature history can be visually as well as semiquantitatively evaluated,
(c) an indicator capable of recording a history from its start, irrespective of its storage conditions, and
(d) to be less expensive and simple in operation so as to be usable by any one.

The temperature history indicator of the present invention by attaching it to food packaging containers is useful for the management as well as the maintenance of food sanitation.

Enzymes, according to the present invention, will now be described.

As enzymes according to the present invention, usable are those originated from microorganisms, animals, and plants. However, enzymes originated from microorganisms are preferred. Further, artificially modified enzymes may be used. Listed as such enzymes are diaholase and peroxydase which promote dye formation through redox reaction, β-galactosidase, alkaline phosphatase, and esterase which promote dye releasing through hydrolysis, and the like.

Preferred enzymes, during enzyme reaction, exhibit maximum activity at about 35 °C, and no activity below 5 °C as well as at at least 65 °C. Enzymes may be added in the form of powder (freeze-dried) or may be added to a buffer solution.

In the present invention, a temperature history indicator itself preferably exhibits high storage stability. Accordingly, enzymes having high temperature stability are preferably employed.

For the purpose of improving the temperature stability of enzymes, said enzymes may be fixed in a material. Listed as such materials for fixing enzymes are nonwoven fabrics, fine particle beads, gels, and the like. Either purified or crude enzymes may be employed.

Microorganisms according to the present invention will now be described.

Listed as microorganisms employed as reagents of the temperature history indicator of the present invention are living microorganisms such as lactic acid bacteria, yeast, and colon bacilli. However, from the viewpoint of food sanitation, it is preferable to use microorganisms such as lactic acid bacteria, yeast (for example, beer yeast and the like) which are perfectly safe for human body.

Further, when microorganisms are used, growth of said organisms is temperature dependent and further, the amount of enzymes produced by said microorganism increases with said growth. Thus, the increased enzymes and the enzyme substrate undergo color reaction which is employed as the temperature history indicator. When microorganisms are used, an enzyme substrate may be either added or not added. When the enzyme substrate is incorporated into the medium, the enzyme substrate is usually not be added.

Further, it was noted that during growth of microorganisms, metabolic substances of said microorganisms resulted in pH variation around said microorganisms. The resulting pH variation can be utilized as a temperature history indicator.

Furthermore, in order to more effectively utilize said pH variation as the temperature history indicator, it is preferable that microorganisms are inactivated, employing a means such as freeze-drying, and it is preferably prepared in the embodiment, in which a medium which becomes nutrients, are separated from said microorganism.

The enzyme substrate according to the present invention will now be described.

The enzyme substrates according to the present invention are determined depending on the selection of enzymes, and are employed from those which form color dyes through reaction with the enzymes. Further, generally utilized may be those employed as clinical test reagents and those used in enzyme immunity measurement methods. Said enzyme substrates may be incorporated into a buffer solution at a preferred concentration. Cited as enzyme substrates of enzymes described above are NADH and tetrazolium salts with respect to diaphorase, benzidines and naphthols with respect to peroxydase, indolylgalactopyrancides with respect to β-galactosidase, indole phosphates with respect to alkaline phosphatase, fluorescein esters with respect to esterase, and the like.

When enzymes are incorporated into a buffer solution, an enzyme substrate may be added in the form of powder. Said enzymes as well as said enzyme substrate is separately arranged. A plurality of enzymes as well as a plurality of enzyme substrates may be employed. When a plurality of enzymes as well as a plurality of enzyme substrates, which are temperature dependent, are employed, it is possible to obtain different hue variations based on temperature history. On the other hand, when a single enzyme as well as a single enzyme substrate is employed, a single dye is formed through an enzyme reaction with respect to temperature history, and detection may be carried out utilizing an increase in the dye density. Dyes having a different hue from that of the formed dye may be previously incorporated so that detection is carried out employing hue variations in addition to the density increase.

For example, a blue dye is previously incorporated in an enzyme substrate composition. When an enzyme which forms a red dye from the start of the temperature history, in the course of the temperature history, hue variations such as blue, violet and red occur and the temperature history can be visually observed.

The pH indictors according to the present invention will now be described.

Employed as pH indicators may be conventional pH indicators known in the art. For example, water-soluble dyes, which result in variations of light absorption wavelength depending on the pH of their surroundings, are suitable. Listed as specific examples of such dyes are 2,6-dinitrophenol, Methyl Yellow, Bromophenol Blue, Bromophenol Red, Congo Red, Methyl Orange, Alizarin S, Bromocresol Green, Methyl Red, Chlorophenol Red, p-nitrophenol, Bromocresol Purple, Bromothymol Blue, Phenol Red, Cresol Red, m-Cresol Purple, Tropaeolin, Thymol Blue, phenolphthalein, and the like.

Further, pH indicators, which are primarily employed in the acidic region, include Thymol Blue (TB, having the color variation range of pH 1.2 to 2.8), Dimethyl Yellow (DY, having a color variation range of pH 2.9 to 4.0), Methyl Orange (MO, having the color variation range of pH 3.1 to 4.4), Bromophenol Green (BPG, having the color variation range of pH 3.0 to 4.6), Bromocresol Green (BCG, having the color variation range of pH 3.8 to 5.4), Methyl Red (MR, having the color variation range of pH 4.2 to 6.3), Bromothymol Blue (BTB, having the color variation range of pH 6.0 to 7.6), Tetrabromophenol Blue, and the like.

By contrast, indicators, which are primarily employed in the alkaline region, include Phenol Red (PR, having the color variation range of pH 6.8 to 8.0), phenolphthalein (PP, having the color variation range pH 8.2 to 10.0), thymolphthalein (TP, having the color variation range of pH 9.3 to 10.5), Alizarin Yellow (AY, having the color variation range of 10.1 to 12.1), and the like.

In the present invention, said pH indicators may detect hydrolysis of the enzyme utilized as the reagent in the temperature history indicator, or may detect the pH variations of the surroundings which vary in accordance with the metabolism of microorganism utilized as the reagent. Thus, pH indictors, which are employed in either an alkaline region or an acidic region, may be employed. However, when beer yeast is employed as the enzyme, or lactic acid is employed as the microorganism, the surroundings become acidic. Accordingly, pH indictors, which work in the acidic region, are preferably employed.

The amount of pH indicators used in the present invention is preferably in the range of 0.01 to 1 percent by weight with respect to the microorganism, and is more preferably in the range of 0.01 to 0.5 percent by weight.

The container of the temperature history indicator of the present invention, in which enzymes as well as enzyme substrates are used as reagents, is characterized in having a function which blends both at the beginning of recording temperature history. In order to blend both an enzyme reagent and an enzyme substrate reagent, listed are means described below:
(a) Either an enzyme or an enzyme substrate is incorporated into a film sheet. At the start of recording temperature history, the cover sheet of said film is peeled off and either the enzyme reagent or the enzyme substrate reagent in a liquid state is dripped onto the exposed area.
(b) An enzyme as well as an enzyme substrate is incorporated into a film of a separate layer. At the start of recording temperature history, moisture is incorporated so that an enzyme reaction is initiated. By contrast, a film is previously arranged to separate the enzyme from the enzyme substrate, and can be peeled away at the start of said reaction.
(c) An enzyme is separated from an enzyme substrate employing a membrane. By pressing one of the reagent housing portion, said membrane is broken to allow both reagents to blend.
(d) An enzyme reagent and an enzyme substrate reagent are separately placed into separate portions connected by a tube which is sealed on at least one end. Under such an arrangement, the sealing of said tube is broken by pressing so that both reagents are blended.
(e) Either an enzyme or an enzyme substrate is housed in microcapsules. At the start of recording tempera rue history, microcapsules are broken by pressing so that both reagents are blended.
(f) Either an enzyme or an enzyme substrate is prepared as the form of a liquid reagent. Both reagents are housed in a film molded container employing heat sealing (so-called blister packaging). At the start of recording temperature history, pressure is applied so that the boundary portion separating both reagents, which has been weakly adhered, is peeled away and both reagents are blended.

The aforementioned means may be applied not only to the container of the temperature history indicator of the present invention in which enzymes as well as enzyme substrates are employed as the reagent, but may also be employed in a case in which enzymes as well as pH indicators are used as the reagents, in a case in which microorganisms as well as enzyme substrates are used as the reagents, in a case in which microorganisms as well as pH indictors are used as the reagents, or the like.

Further, the present invention is not limited to the aforementioned means.

The temperature range, in which the temperature history indicator of the present invention is employed, will now be described.

In the temperature history indicator of the present invention, a reaction between an enzyme and an enzyme substrate is a color forming reaction. Said reaction preferably proceeds in the range of 0 to 80 °C, and more preferably proceeds in the range of 5 to 65 °C.

The temperature history indicator is employed for the temperature management during distribution and storage of food, and the like. The objective of temperature management related to food is to retard the growth of microorganisms (bacteria). Namely it is important to avoid the temperature range of 5 to 65 °C, which is suitable for the growth of bacteria. Specifically, the active growth occurs in the temperature range of 20 to 40 °C. Bacteria are in a dormant state at no more than 4 °C and thus do not grow. Further, even in the temperature range of about 50 to 65 °C, bacteria are in a dormant state and generally are killed at over 75 °C. It is known that there are exceptional bacteria which are not killed even over 100 °C. However, in such a case, bacteria do not grow.

It is understood that the temperature history indictor of the present inventions is adhered onto food packaging materials, for example, a corrugated cardboard box. Thus, the configuration is preferred which bears adhesive agents such as tapes or seals on the opposite surface. Further, it is possible to provide a blank on which the initiation time for recording temperature history can be written.

### EXAMPLES

Examples of the present invention will now be described. However, the present invention is not limited to the examples.

### Example 1

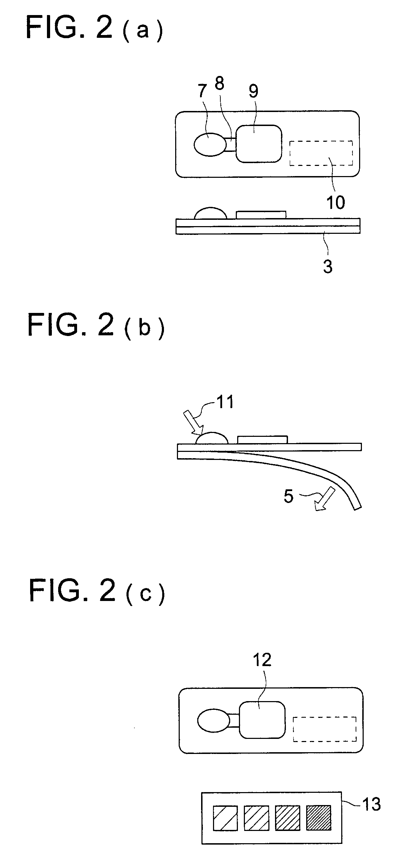

As described below, prepared was the blister packaging container type temperature history indicator as shown in Fig. 2(a).

### <<Preparation of Blister Packaging Container Type Temperature History Indicator >>

A 0.3 mm thick polypropylene film was molded at 220 °C. A polyethylene coating agent having a low softening point was applied to the boundary portion of two concave areas, and weakly adhered portions were prepared. In one of two concave areas, 0.3 ml of an enzyme substrate liquid was provided and in the other concave, a piece of nonwoven fabric was provided. The resultant molded film was covered with a 0.1 mm thick white polypropylene film, which was then subjected to heat adhesion at 22 °C for 5 seconds. The surface of said white polypropylene was adhered with a double coated adhesive tape. Further, a 2 × 6 cm outer size temperature history indicator was punched out.

### <<Preparation of the Enzyme Substrate Liquid>>

Dissolved in 5 ml of distilled water was 1 g of 4-methylumbellifery phosphate·2Na. Dissolved in 500 ml were 10 g of table salt, 2 g of sodium bicarbonate, and 50 mg of Thymol Blue, and the pH of the resultant mixture was adjusted to 9 by using an aqueous 1 mole/liter sodium hydroxide solution. Said substrate and said buffer solution were blended, whereby a enzyme substrate liquid was prepared.

### <<Preparation of the Enzyme Containing Nonwoven Fabric>>

Dissolved in 5 ml of physiological saline solution were 5 mg of freeze-dried powder of alkaline phosphatase (originated from cow pancreas, 500 unit/mg), and the resultant solution was further diluted by a factor of 1000, employing a physiological saline solution. A 2 mm thick nonwoven fabric (ES fiber, manufactured by Nihon Bilean Co.) was cut to 1 cm², and 100 µl of said enzyme liquid was dripped onto it and subsequently freeze-dried.

### <<Results of Temperature History Tests>>

As shown in Fig. 2(b), substrate solution containing part 11 of the blister packaging container type temperature history indicator was broken by pressing and enzyme containing nonwoven fabric 12 was impregnated with said substrate solution. Subsequently 100 g of ground meat which were in the form of two packages were purchased on the open market. Each package was pasted with said blister packaging container type temperature history indicator and was stored in either a 35 °C incubator or a 4 °C refrigerator. Along with the elapse of time described below, the number of bacteria in 1 g of said ground meat was measured in the agar culture medium for common bacteria. Further, as shown in Fig. 2(c), the temperature variation of color of the temperature history indicator was visually observed. Table 2 shows the correlation between the temperature history of the temperature history indicator and the number of bacteria.

**Table 2**

| Storage Time | Sample Stored at 4 °C | | Sample Stored at 35 °C | |
|---|---|---|---|---|
| | Number of Bacteria | Color of Temperature History Indicator | Number of Bacteria | Color of Temperature History Indicator |
| At Beginning | 0.9 × 10³ | pale blue | 1.5 × 10³ | pale blue |
| After 3 Hours | - | pale blue | 4.8 × 10³ | pale blue |
| Hours 6 Hours | - | pale blue | 7.3 × 10⁴ | pale violet |
| After 12 Hours | | pale blue | 2.8 × 10⁶ | reddish violet |
| After 24 Hours | 1.2 × 10³ | pale blue | 1.1 × 10⁸ | red |
| After 48 Hours | 5.5 × 10³ | pale violet | 3.5 × 10⁸ | reddish brown |

As can be seen from Table 2, from the beginning to after 48 hours during the storage at 4 °C, the increase in the number of bacteria was minor, and the color variation of the temperature history indicator was also minor. By contrast, the number of bacteria in the sample after 48 hours, which was stored at 35 °C, increased by a factor of about 100,000, and corresponding to said increase, the color of the temperature history indicator markedly varied from pale blue to reddish brown and exhibits excellent correlation with the growth of bacteria. Based on the fact described above, it is clear that the temperature history indicator, when it is attached to a packaging container of food, is markedly useful for the management and maintenance for food sanitation.

### Example 2

### <<Preparation of Enzyme Substrate Solution>>

After dissolving 0.1 g of 2,3,5-triphenyltetrazolium chloride, 20 g of glucose, 5 g of peptone prepared from casein, 0.5 g of magnesium sulfate, 1 g of potassium hydrogen phosphate, 0.03 g of tetracycline, and 1 mg of potassium benzyl penicillin in 1000 ml of distilled water, the resultant solution was filtered with a 0.22 µm pore filter and then bacteria were removed.

### <<Preparation of Yeast>>

After dissolving 1 g of dried bread yeast powder in 100 ml of distilled water, the resultant solution was filtered through a 0.22 µm pore filter and bacteria were removed. Onto a nonwoven fabric, 100 µl of said yeast solution was dripped and then freeze dried.

### <<Preparation of Blister Package>>

Blister packaging was carried out in the same manner employing the method described in Example 1, and 0.3 ml of the enzyme substrate solution, as well as the yeast containing nonwoven fabric was loaded.

### <<Results of Temperature History Test>>

A color sample, which was employed to evaluate the degree of color formation of the temperature history indicator of the present invention, was prepared as described below.
No coloration: the temperature history management is acceptable
Pink: care is required
Red: the temperature management history is not acceptable.

The temperature history indicator of the present invention was attached onto the opposite lid of the corrugated carton in which a sandwich was placed, and was stored in condition (1) of 4 °C and 24 hours, condition (2) of room temperature (20 °C) and 12 hours, and condition (3) of room temperature (20 °C) and 24 hours.

It was observed that the sample stored at 4 °C resulted in no coloration of the temperature history indicator of the present invention, and the sample stored at room temperature for 12 hours resulted in pink indication, and the sample stored at room temperature for 24 hours resulted in red indication.

As described above, it was found that the temperature history indicator of the present invention was capable of recording the history in the specified temperature range as well as in the specified time range.

### Example 3

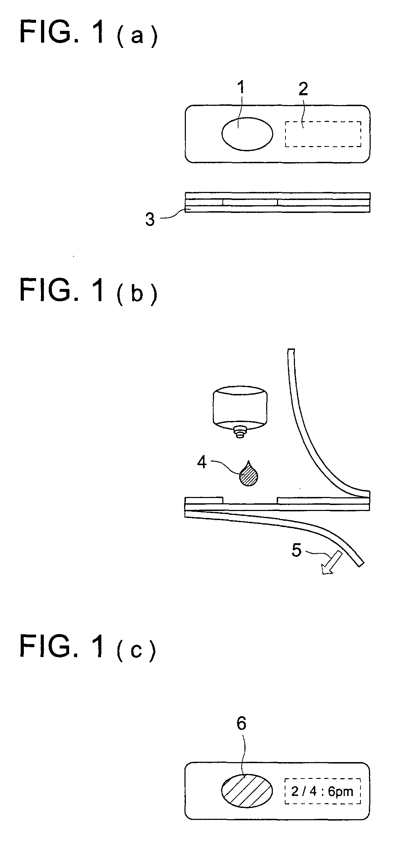

A development sheet was prepared as described below. Subsequently the enzyme substrate liquid described below was dripped onto an enzyme reagent development area, whereby a temperature history indicator prior to use was prepared. Fig. 1(a) shows the described indicator.

### <<Preparation of a Development Sheet>>

A 0.3 mm thick white PET film was cut to 2 × 5 cm, and a 2 cm wide double coated adhesive tape was adhered onto one side. A 2 mm thick polypropylene board was also cut to 2 × 5 cm. A 1 × 2 cm elliptical hole was made which was employed for an enzyme agent development area, and was adhered onto said PET film employing an adhesive.

### <<Preparation of the Enzyme Substrate Liquid>>

An enzyme substrate liquid was prepared as described below.

Three mg of X-Gal (5-bromo-4-chloro-3-indolyl-β-D-galactopyranoside) was dissolved in 1 ml of DMF (dimethylformamide). After dissolving 10 g of table salt and 1.5 g of sodium hydrogenphosphate in 700 ml of distilled water, the pH was adjusted to 7.5 by using diluted hydrochloric acid. Added into 50 ml of the resultant buffer solution was 0.1 ml of an X-Gal solution, to prepare the enzyme substrate liquid. Dripped onto said development sheet was 0.2 ml of said enzyme substrate liquid, and the resultant sheet was allowed to dry at room temperature. The upper surface of the dried sheet was sealed with a transparent vinyl tape.

### <<Preparation of Enzyme Liquid>>

Dissolved in 1 ml of a physiological saline solution were five mg of freeze-dried product of β-galactosidase (originated from colon bacilli, 500 units/mg). The resultant solution was further diluted by a factor of 100, employing a physiological saline solution. The resultant solution was stored at 4 °C. Dissolved in 500 ml of distilled water were 10 g of table salt, 1.5 g of sodium monohydrogen phosphate, and 200 g of glycerin. Employing diluted hydrochloric acid, the pH of the resultant solution was adjusted to 7.2. Added to 50 ml of the resultant buffer solution were 100 µl of the diluted solution of said enzyme solution to obtain an enzyme liquid. A drip bottle was then filled with the resultant enzyme liquid.

### <<Test Results of Temperature History>>

As shown in Fig. 1(b), the vinyl tape of the aforementioned development sheet was peeled away. After dripping 4 drops of said enzyme liquid from said drip bottle, the upper surface was resealed with the vinyl tape and three samples as one of which is shown in Fig. 1(c), were prepared. Each sample was adhered onto the wall of three thermostats set at 4 °C, 37 °C, and 65 °C. After an elapse of specified time in the thermostat, the reflection density at 630 nm was measured. Table 1 shows the results.

**Table 1**

| Time | Density of Sample Stored at 4 °C (at 630 nm) | Density of Sample Stored at 37 °C (at 630 nm) | Density of Sample Stored at 65 °C (at 630 nm) |
|---|---|---|---|
| Start | 0.06 | 0.04 | 0.06 |
| After 1 Hour | 0.05 | 0.23 | 0.14 |
| After 2 Hours | 0.08 | 0.37 | 0.11 |
| After 5 Hours | 0.07 | 0.67 | 0.14 |
| After 8 Hours | 0.12 | 1.50 | 0.22 |
| After 12 Hours | 0.10 | 2.49 | 0.14 |
| After 24 Hours | 0.15 | 2.38 | 0.20 |

As can be seen from Table 1, the sample of the present invention resulted in an increase in blue density over an elapse of time at 37 °C, while at 4 °C and 65 °C, the samples resulted in almost no increase in blue density and thus the temperature history indicator of the present invention shows it to be capable of definitely recording temperature history at a specified temperature.

The present invention is capable of providing a disposable temperature history indicator which can be directly attached onto a food package and is useful for the management as well as the maintenance of food sanitation.

Disclosed embodiment can be varied by a skilled person without departing from the spirit and scope of the invention.

## Claims

1. A temperature history indicator comprising an enzyme or a microorganism and an enzyme substrate and a container to contain the enzyme or the microorganism and the enzyme substrate,
wherein the enzyme substrate is separated from the enzyme or the microorganism,
the enzyme substrate reacts with the enzyme or the microorganism by mixing the enzyme substrate with the enzyme or the microorganism,
a specific color is created in response to the reaction, and the reaction proceeds at the specified temperature range.

2. The temperature history indicator of claim 1, wherein the reaction proceeded by mixing the enzyme substrate with the enzyme or the microorganism is color creating reaction.

3. The temperature history indicator of claim 1, wherein the temperature history indicator comprises a pH indicator, the reaction proceeded by mixing the enzyme substrate with the enzyme or the microorganism is a reaction to induce pH variation, and the pH indicator creates color in response to the pH variation.

4. The temperature history indicator of claim 1, wherein the specified temperature range falls within the range of 0 to 80 °C.

5. The temperature history indicator of claim 4, wherein the specified temperature range falls within the range of 4 to 65 °C.

6. The temperature history indicator of claim 1, wherein the enzyme is a thermoduric enzyme which is not modified at 50° C or the microorganism is a thermoduric microorganism which does not perish at 50° C.

7. The temperature history indicator of claim 1, wherein the enzyme is an enzyme derived from a thermophilic bacterium or the microorganism is a thermophilic bacterium.

8. The temperature history indicator of claim 7, wherein the enzyme is alanine dehydrogenase derived from bacillus stearothermophilus, alanine racemase derived from bacillus stearothermophilus, or formate dehydrogenase derived from a microorganism.

9. The temperature history indicator of claim 7, wherein the microorganism is bacillus stearothermophilus.

10. The temperature history indicator of claim 1, wherein the container comprises a first containing portion containing the enzyme or the microorganism and a second containing portion containing the enzyme substrate, and the container constituted so that the enzyme or the microorganism can be mixed with the enzyme substrate by pressing the first or second containing portion.

11. The temperature history indicator of claim 10, wherein the first containing portion or the second containing portion is visibly observable.

12. The temperature history indicator of claim 10, wherein the enzyme or microorganism is dissolved or dispersed in a liquid.

13. The temperature history indicator of claim 10, wherein the enzyme substrate is dissolved or dispersed in a liquid.

14. The temperature history indicator of claim 10, wherein the temperature history indicator comprises a adhesive portion by which the temperature history indicator is adhered on another thing.
